Europäisches Patentamt

European Patent Office

Office européen des brevets

(19)

(11) Publication number: **0 217 621**
A2

(12)

# EUROPEAN PATENT APPLICATION

(21) Application number: 86307303.7

(22) Date of filing: 23.09.86

(51) Int. Cl.⁴: **C 12 P 1/06**
C 07 G 11/00, C 12 N 1/20
A 61 K 35/66
//C12R1/03, (C12P1/06,
C12R1:01, C12N1:20, C12R1:01)

(30) Priority: 27.09.85 US 781292

(43) Date of publication of application:
08.04.87 Bulletin 87/15

(84) Designated Contracting States:
AT BE CH DE FR GB IT LI LU NL SE

(71) Applicant: SMITHKLINE BECKMAN CORPORATION
P.O. Box 7929 1 Franklin Plaza
Philadelphia Pennsylvania 19101(US)

(72) Inventor: Chan, James Amigo
1215 Uppon Circle
West Chester Pennsylvania 19380(US)

(72) Inventor: Johnson, Randall Keith
71 Llanfair Circle
Ardmore Pennsylvania 19002(US)

(74) Representative: Waters, David Martin, Dr. et al,
Smith Kline & French Laboratories Ltd. Patent
Department Mundells
Welwyn Garden City Hertfordshire AL7 1EY(GB)

(54) Macromolecular tumor cell growth-inhibiting antibiotic.

(57) This invention relates to a novel macromolecular tumor cell growth-inhibiting antibiotic; the microorganism which produces such antibiotic; a pharmaceutical composition comprising an effective, tumor cell growth inhibiting amount of such antibiotic and to its use in treating tumor cells in a host animal afflicted by such tumor cells.

EP 0 217 621 A2

Croydon Printing Company Ltd

MACROMOLECULAR TUMOR CELL GROWTH-INHIBITING ANTIBIOTIC

This invention relates to a novel macromolecular tumor cell growth-inhibiting antibiotic, and to the actinomycete which produces such antibiotic. This invention also relates to a pharmaceutical composition comprising an effective, non-toxic, tumor cell growth-inhibiting amount of such antibiotic and an inert pharmaceutically acceptable carrier or diluent, and to the use of the antibiotic in treating tumor cells in a host animal afflicted by such tumor cells.

This invention relates to a macromolecular tumor cell growth-inhibiting and cytotoxic antibiotic compound, AAC-345, which has the following characteristics:

| | |
|---|---|
| Nature: | White amorphous powder |
| Solubility: | Soluble in water |
| Stability: | Sensitive to light in aqueous solution, stable as lyophilized powder |
| Molecular Weight: | ~42,000 by SDS-PAGE |
| Isoelectric Point: | pI = 2.9 |
| Carbohydrate Content: | 12% by phenol/sulfuric acid method |
| HPLC: | Retention time, 11.5 min; TSK-3000 SW Column (7.5 mm x 30 cm); Mobile Phase, 0.05M potassium phosphate buffer at pH 6.3; flow rate, 1 ml/min; detection, 220 nm and 280 nm |
| UV $\lambda_{max}$ ($E_{1cm}^{1\%}$ in $H_2O$): | 220 nm (21.6); 275 nm (3.0) |

Amino Acid Analysis:      Asp (2862.4), Glu (1524.4),
    (Picomole)            Ser (1135.8), Gly (1504.9),
                          His (41.3), Arg (107.4), Thr
                          (2097.8), Ala (1765.7), Pro
                          (793.8), Tyr (134.5), Val
                          (1822.8), Met (223.9), Ile
                          (456.3), Leu (307.0), Phe
                          (298.2), Lys (49.4);

Partial Amino Acid        N-Asp-Thr-Val-Thr-Val
Sequence:

This invention also relates to a biologically pure culture of an actinomycete microorganism which has the identifying characteristics of ATCC 53270, and active mutants and derivatives thereof.

This invention also relates to a pharmaceutical composition which comprises an effective, non-toxic, tumor cell growth-inhibiting amount of AAC-345 and an inert pharmaceutically acceptable carrier or diluent.

The macromolecular tumor cell growth-inhibiting antibiotic of this invention, AAC-345, can be prepared as a crude broth, enriched crude broth or a purified product from the fermentation broth of an unidentified actinomycete (probably an _Actinomadura_ sp) which was deposited on September 20, 1985 with the American Type Culture Collection, Rockville, Maryland, U.S.A. under the accession number ATCC 53270. As used herein, the term "crude broth of ATCC 53270" means the clarified fermentation broth of ATCC 53270 such as the filtrate remaining after filtration or centrifugation of the fermentation broth by conventional techniques. As used herein the term "enriched crude broth of ATCC 53270" means the clear filtrate remaining after further filtration of the clarified crude broth of ATCC 53270 by

-3-

0217621

ultracentrifugation or diafiltration or other procedure including chromatographic separation. See, e.g., Example 3, below. As used herein the term "purified active component of ATCC 53270" means the substance remaining after purifying the crude broth of ATCC 53270 to a purity of at least 95% by any appropriate procedure, such as the method described in Example 3. Derivatives of AAC-345 can be prepared by known techniques and all active derivatives of AAC-345 are included within the scope of the specification. By the term "active derivatives of AAC-345" is meant those derivatives of AAC-345 which substantially retain the tumor cell growth-inhibiting activity of AAC-345. Such derivatives include, but are not limited to, derivatives prepared by the addition, deletion or substitution of any of the amino acids comprised by AAC-345, which substantially retain the tumor cell growth-inhibiting activity of AAC-345; and derivatives which are complexes of AAC-345 with other compounds or molecules wherein said complexes substantially retain the tumor cell growth-inhibiting activity of AAC-345.

The microorganism of this invention, referred to hereafter as ATCC 53270, when cultivated under appropriate conditions, produces a fermentation broth which will be referred to hereafter as the fermentation broth of ATCC 53270. By the term "cultivated under appropriate conditions" is meant those conditions which enable the growth of ATCC 53270 and its production of the fermentation broth of ATCC 53270. Illustrative appropriate conditions include growing ATCC 53270 in an aqueous nutrient medium containing assimilable sources of carbon and nitrogen under submerged aerobic conditions at a temperature between 25° to 30°C, preferably 28°C, at a pH between 7 and 8 for 10 to 100 hours, preferably 50 to 80 hours, most preferably from 70 to 77 hours. More preferably, the appropriate conditions for cultivation of ATCC 53270 to produce the fermentation broth of ATCC 53270 are substantially similar to the conditions described in Example 1. It will be understood by one of skill in the art that the appropriate conditions of cultivating ATCC 53270 can be optimized for production of AAC-345.

This invention relates to a biologically pure culture of an actinomycete microorganism which has the identifying characteristics of ATCC 53270, and active mutants and derivatives thereof. By "active mutants and derivatives" is meant mutants or derivatives of ATCC 53270 which retain the ability to produce a fermentation broth containing the antibiotic of the subject invention. Such mutants and derivatives can be prepared by known techniques, for example, by chemical mutagenesis, recombinant DNA techniques, irradiation and selection.

As stated above, the antibiotic of this invention, AAC-345, has cytotoxic activity. Initially, the cytotoxic activity of the crude broth of ATCC 53270 and the purified active component of ATCC 53270 were evaluated _in vitro_ using B16 melanoma cells according to the following assay [see, Mirabelli et al., _J. Antibiotics_, _38_, 758-766 (1985)]:

B16 melanoma (highly metastatic subline, F10) are used and maintained as monolayer cultures in Minimal Essential Media (Grand Island Biological Co., Grand Island, N.Y.) supplemented with 10% calf serum, 1% antibiotics in a 5% $CO_2$ humidified incubator at 37°C. Asynchronous populations of cells are harvested and replated to 5000 cells/plate in sterile 60 mm petri plates. Plates are incubated overnight to allow attachment of the cells to the plate. Cells are treated with the crude broth of ATCC 53270 or the purified active component of ATCC 53270 under sterile conditions, allowed to react for 1 or 24 hours followed by aspiration of medium. Plates are washed one time with 5 ml of phosphate buffered saline (PBS), followed by aspiration of medium. Plates are incubated for 5 days at 37° in a $CO_2$ incubator with fresh medium and serum. Viability is measured by the ability of a cell to form a colony of greater than 50 cells. Colonies are fixed with 0.5% crystal violet in 95% ethanol. Plates are dried and counted with a Biotran III Automatic Count Totalizer (New Brunswick Scientific Co., Edison, N.J.). Mean and standard deviation of triplicate samples are determined for each drug concentration. The data are analyzed by plotting the log of the survival fraction (number of colonies in drug treated plates/number of colonies in controls) versus the broth dilution or drug concentration. The 50 percent inhibitory concentration ($IC_{50}$) is determined from this plot.

AAC-345, in the form of purified active component of ATCC 53270, reduced the viability of B16 cells by 50 percent in the _in vitro_ B16 melanoma assay at a concentration of 0.56 μg/ml when cells were exposed to the active component for 1 hour. For a 24-hour exposure, the 50 percent cytotoxic concentration was approximately 0.01 μg/ml. Thus, the antibiotic of the subject invention is a potent cytotoxic agent for tumor cells.

As stated above, AAC-345, the antibiotic of this invention has tumor cell growth-inhibiting activity which has been demonstrated in a number of animal tumor models.

P388 lymphocytic leukemia is currently the most widely used animal tumor model for screening for antitumor agents and for detailed evaluation of active compounds. This tumor system is widely accepted as an antitumor agent screening tool because it is sensitive to virtually all of the clinically active antineoplastic agents; quantitative and reproducible; amenable for large-scale screening; and predictive for activity in other animal tumor models. Drugs that are highly active in the intraperitoneal (ip) P388 leukemia model are generally active in other tumor models as well. The anti-tumor activity of AAC-345, in the form of the crude broth of ATCC 53270 and the purified active component of ATCC 53270, was demonstrated in the P388 leukemia mouse model employing the following protocol [see, Geran et al., Cancer Chemother. Rpts. (Part III), 3, 1-103 (1972)]:

$10^6$ P388 leukemia cells are inoculated ip in B6D2F$_1$ mice. Twenty-four hours later, if the tumor inoculum proves to be free of bacterial contamination (as determined by 24 hours incubation in thioglycollate broth), animals are randomized into groups of 6 and housed in shoebox cages. The purified active component of ATCC 53270 was dissolved in 0.9 percent NaCl solution (normal saline) at an appropriate concentration so that the desired dose was delivered in a volume of 0.5 ml. The crude broth of ATCC 53270 was administered undiluted or diluted with normal saline. Such saline solutions were stored in amber bottles at 4°C. The saline solution of the purified active component of ATCC 53270, saline solution of the crude broth of ATCC 53270 or undiluted crude broth of ATCC 53270 are administered ip on Days 1 through 5 (i.e. treatment is initiated 24 hrs after tumor inoculation). Each experiment includes three groups of 6 animals as untreated controls and animals treated with a positive control, cisplatin. Animals are weighed as a group on Days 1, 5 and 9 and average weight change ($\delta$ wt.) is used as a reflection of toxicity. Each experiment also includes an inoculum titration -- groups of 8 mice inoculated ip with $10^5$ to $10^0$ P388 leukemia cells. The titration is used to calculate cell kill achieved by treatment with drugs. Animals are monitored daily for mortality and experiments are terminated after 45 days. The endpoint is median survival time (MST) and increase in lifespan (ILS) which is the percentage of increase in MST relative to untreated controls. Untreated controls inoculated ip with $10^6$ P388 leukemia cells generally survive for a median of 9 to 11 days. A drug is considered active if it produces $\geq$ 25 percent ILS.

A summary of the evaluation of the purified active component of ATCC 53270 and the crude broth of ATCC 53270 in the _in vivo_ ip P388 model is shown in the following Table A.

## TABLE A

| Preparation | Dilution of crude broth | Dose of pure material mg/kg/day) | Weight Change (gm) Day 5 | Weight Change (gm) Day 9 | Median Survival Time (days) | ILS[a] (%) |
|---|---|---|---|---|---|---|
| **Experiment I** | | | | | | |
| Crude broth of ATCC 53270 | Undiluted | | dead | | 3.5 | toxic |
| | 1:2 | | dead | | 4 | toxic |
| | 1:4 | | -2.8 | dead | 7 | toxic |
| | 1:8 | | -1.6 | -2.8 | 14.5 | 56 |
| | 1:16 | | -1.0 | +0.4 | 14.5 | 61 |
| | 1:32 | | -1.0 | +3.7 | 14.5 | 61 |
| | 1:64 | | 0 | +3.9 | 14.5 | 61 |
| | 1:128 | | +1.1 | +4.1 | 13 | 44 |
| | 1:256 | | +1.4 | +3.1 | 12 | 33 |
| Cisplatin | | 1 | -0.2 | +1.4 | 19.5 | 117 |
| Untreated Controls | | 0 | +2.3 | +4.4 | 9 | - |
| **Experiment II** | | | | | | |
| Purified Active Component of ATCC 53270 | | 4 | -2.9 | dead | | toxic |
| | | 2 | -2.1 | -4.3 | 16 | toxic |
| | | 1 | -1.8 | -1.6 | 19 | 111 |
| | | 0.5 | -1.1 | -0.4 | 20 | 122 |
| | | 0.25 | -0.5 | +0.4 | 20.5 | 128 |
| | | 0.125 | 0 | +1.6 | 18 | 100 |
| | | 0.062 | +0.6 | +1.9 | 15.5 | 72 |
| | | 0.031 | +1.2 | +4.2 | 14.5 | 61 |
| | | 0.016 | +1.2 | +3.6 | 14 | 56 |
| Cisplatin | | 1 | -0.6 | +1.2 | 17 | 89 |
| Untreated Controls | | 0 | +2.8 | +4.0 | 9 | - |

(a) increase in lifespan produced in mice bearing ip P388 leukemia.

Based on the data set forth in Table A, AAC-345, in the form of the crude broth of ATCC 53270 and the purified active component of ATCC 53270, showed significant antitumor activity in the _in vivo_ ip P388 leukemia tumor assay at all non-toxic doses.

Likewise, AAC-345, in the form of the crude broth of ATCC 53270 and the purified active component of ATCC 53270, was tested in an additional _in vivo_ tumor model known as ADJ-PC6 Plasmacytoma according to the following assay [see Cleare et al., _J. Biochimie_, _60_, 830-850 (1978)]:

Tumor cells are carried by serial sc passage in BALB/c female mice and then collected aseptically on ca. Day 21 and minced in Hank's balanced salt solution. The cells are then dispersed by homogenizer, and cell concentration is adjusted to $4 \times 10^6$ viable (trypan blue-excluding) cells per ml by hemocytometer counts. A total of 0.5 ml ($2 \times 10^6$ cells) is implanted sc on the right flank of BALB/c female mice in groups of 8. Treatment is given ip on Days 1-10, and tumors are measured in perpendicular diameters with a vernier caliper approximately three (3) weeks after tumor implantation (Days 18-21). Generally, $\geq 75\%$ inhibition of tumor growth reflects significant antitumor effect. Cisplatin, the positive control compound, produces complete tumor growth inhibition.

The results of the ADJ-PC6 Plasmacytoma assay are summarized in Table B.

## TABLE B

| Preparation | Dilution of crude broth | Dose[c] purified material (mg/kg/day) | Weight Change (gm) Day 21 (Expt I) Day 19 (Expt II) | Mean Tumor[b] Volume ($mm^3 \pm$ SEM) | TGI[a] (%) |
|---|---|---|---|---|---|
| | **Experiment I** | | | | |
| Crude Broth of ATCC 53270 | 1:40 | | -2.5 | $141 \pm 25$ | 91 |
| | 1:80 | | +0.4 | $561 \pm 125$ | 66 |
| | 1:160 | | +1.2 | $748 \pm 219$ | 54 |
| | 1:320 | | +2.2 | $1035 \pm 179$ | 37 |
| | 1:640 | | +2.4 | $1046 \pm 233$ | 36 |
| Cisplatin | | 3 | -0.8 | $0 \pm 0$ | 100 |
| Untreated Control | | 0 | +2.4 | $1639 \pm 169$ | |
| | **Experiment II** | | | | |
| Purified Active Component of ATCC 53270 | | 2 | dead | toxic | |
| | | 1 | dead | toxic | |
| | | 0.5 | dead | toxic | |
| | | 0.25 | -2.9 | $0 \pm 0$ | 100 |
| | | 0.125 | -0.8 | $0 \pm 0$ | 100 |
| | | 0.062 | +0.4 | $8 \pm 8$ | 99 |
| | | 0.031 | +1.1 | $83 \pm 19$ | 87 |
| Cisplatin | | 1.5 | -1.5 | $0 \pm 0$ | 100 |
| Untreated Control | | 0 | +2.0 | $662 \pm 61$ | |

(a)  Percent of tumor growth inhibition produced in mice bearing ADJ-PC6 Plasmacytoma.
(b)  Tumors were measured on Day 21 in Experiment I and Day 19 in Experiment II.
(c)  Drug was administered ip on Days 1-10.

Based on the data in Table B, AAC-345, both in the form of the crude broth of ATCC 53270, and, in particular, in the form of the purified active component of ATCC 53270, displayed excellent antitumor activity in the ADJ-PC6 Plasmacytoma assay at all non-toxic doses producing $\geq$ 75% inhibition of tumor growth.

The pharmaceutical compositions of this invention comprise an effective, non-toxic, tumor cell growth-inhibiting amount of the macromolecular tumor cell growth-inhibiting antibiotic of this invention, AAC-345, and an inert pharmaceutically acceptable carrier or diluent. These compositions are prepared in dosage unit form appropriate for parenteral administration.

Compositions according to the invention for parenteral administration include sterile aqueous or non-aqueous solutions, suspensions or emulsions. The composition may be in the form of a solution of the purified active component of ATCC 53270 in a minimal volume of dimethylacetamide or ethanol, for example 5% v/v, brought up to volume with peanut oil or normal saline solution. Polyethoxylated castor oil, for example 2 to 5% v/v, may also be used to solubilize the active ingredient. In addition, the composition may be in the form of a slurry with, for example, hydroxypropyl cellulose or other suitable suspending agent. As an emulsifying agent, lecithin for example may be used. The composition may also be provided in the form of a sterile solid which can be dissolved in a sterile injectable medium immediately before use.

It will be appreciated that the actual preferred dosage of the macromolecular antibiotic of this invention used in the compositions of this invention will vary according to the particular composition formulated, the mode of administration and the particular site, host and disease being treated. The route of internal administration should be selected to ensure that an effective tumor cell growth-inhibiting amount of the antibiotic of this invention contacts the tumor. Optimal dosages for a given set of conditions can be ascertained by those skilled in the art using conventional dosage determination tests in view of the above experimental data. For parenteral administration, the dose generally employed is from about 1 to about 10 mg/m$^2$ of body surface per day for one to five days, repeated about every fourth week for four courses of treatment.

The method for inhibiting the growth of animal tumor cells sensitive to the antibiotic of this invention comprises administering internally to a host animal afflicted with said tumor cells, an effective, non-toxic, tumor cell growth-inhibiting amount of the antibiotic of this invention. As described above, during the course of treatment the active ingredient will be administered, preferably parenterally, in an amount selected from about 10 mg to about 100 mg/m$^2$ of body surface.

### EXAMPLES

The following Examples illustrate only one of any number of methods for the preparation of the crude broth of ATCC 53270 and the purified active component of ATCC 53270 which are used in the composition and method of this invention, and as such are not to be construed as limiting the scope thereof.

The following abbreviations are employed in the examples:

1. **MEDIUM 13H**

| | |
|---|---|
| Starch | 15 g/L |
| Sucrose | 5 g/L |
| Dextrose | 5 g/L |
| Hysoy | 7.5 g/L |
| Corn Steep Liquor | 5 g/L |
| $K_2HPO_4$ | 1.5 g/L |
| NaCl | 0.5 g/L |
| Mineral "S" | 5 g/L |
| $CaCO_3$ | 1.5 g/L |

2. **MINERAL "S"**

| | |
|---|---|
| $ZnSO_4 \cdot 7H_2O$ | 2.8 g/L |
| $Fe(NH_4)_2HC_6H_5O_7$ | 2.7 g/L |
| $CuSO_4 \cdot 5H_2O$ | 0.125 g/L |
| $MnSO_4 \cdot H_2O$ | 1.0 g/L |
| $CoCl_2 \cdot 6H_2O$ | 0.1 g/L |
| $Na_2B_4O_7 \cdot 10H_2O$ | 0.09 g/L |
| $Na_2MoO_4 \cdot 2H_2O$ | 0.05 g/L |

### EXAMPLE 1

#### PREPARATION OF THE FERMENTATION BROTH OF ATCC 53270

The entire growth from an agar slant of ATCC 53270 was inoculated into 500 mL of medium 13H in a 4-liter aspirator bottle, and then was incubated at 28°C for 5 days on a rotary shaker (New Brunswick Model G53) at

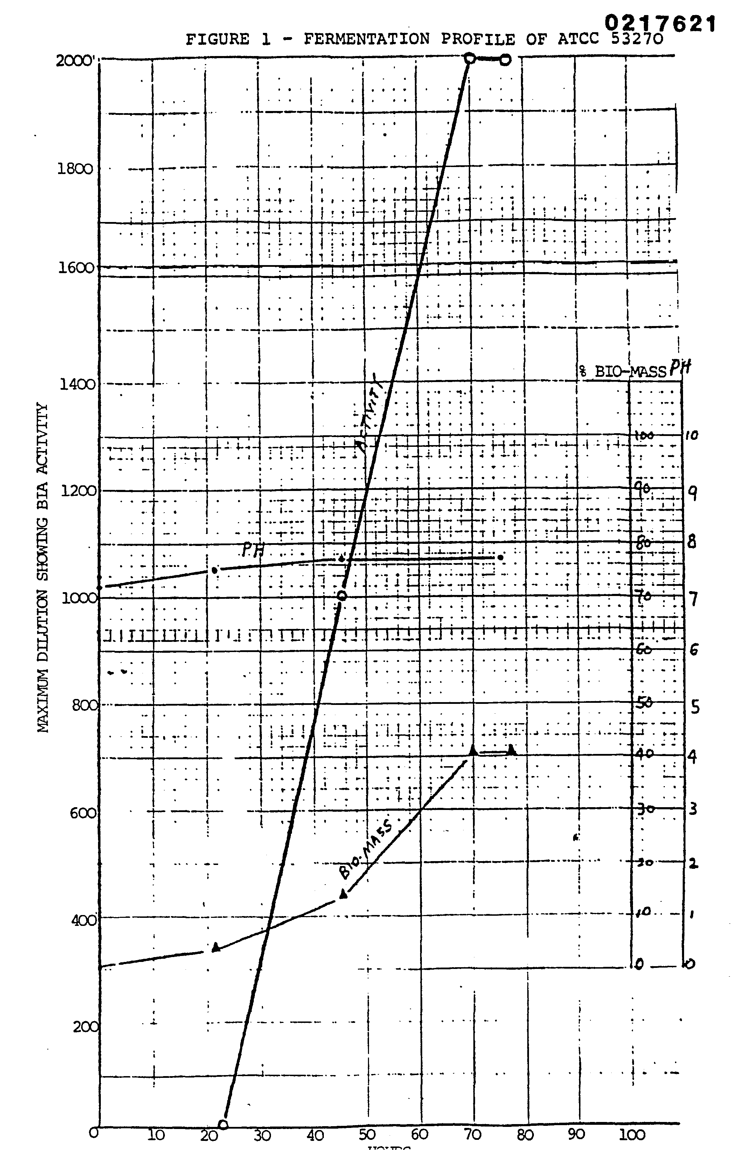

250 RPM and 2 inch (5.08 cm) throw. The 5-day seed was then transferred to a 14-liter fermentor (New Brunswick Model 19) with 10 liters of medium 13H. The fermentation was conducted at 28°C, aerated at 4-liter/minute and agitated at 400 RPM. The fermentation was harvested at 77 hours based on Biochemical Induction Assay (BIA) activity as described by H.F. Bartus et al., Antimicrob. Agents Chemother., 25, 622-625 (1984). Figure 1 is a graph of the fermentation profile of the fermentation broth of ATCC 53270, and compares the growth of ATCC 53270 over time (including pH measurement) and activity of the fermentation broth of ATCC 53270 over time i.e., the highest dilution at which the fermentation broth retains activity in the BIA.

## EXAMPLE 2

### PREPARATION OF THE CRUDE BROTH OF ATCC 53270

The fermentation broth of ATCC 53270, prepared as described in Example 1, was centrifuged at 14,000xG for 15 minutes (min), and the resulting pellet was discarded. The remaining supernatant is the crude broth of ATCC 53270.

## EXAMPLE 3

### PREPARATION OF THE PURIFIED ACTIVE COMPONENT OF ATCC 53270

The crude broth of ATCC 53270, prepared as described in Example 2, was put through an ultrafiltration apparatus (Amicon DC-2) equipped with two 30,000 molecular weight cut-off cartridges, and was concentrated to about 250 ml. The retentate was then diafiltered four times against equal volumes of water. Upon completion of the diafiltration, the retentate was frozen for storage before proceeding to the next purification step. After the frozen retentate was thawed, it was centrifuged for 30 min at 8,000xG to remove any precipitate. The resulting clear retentate is the enriched crude broth of ATCC 53270.

The clear retentate (enriched crude broth of ATCC 53270) was loaded onto a DEAE-Trisacryl M® ion exchange column (LKB, 5 cm inside diameter (ID)x15 cm length) previously equilibrated with 0.05 M Tris-acetate buffer (pH 8.3) at 4°C in the dark. The column was run at a flow rate of 8 ml/min, and elution was followed by UV detection at 220 nm at 2 absorbance unit full scale (AUFS). The column was sequentially eluted with 560 ml of Tris-acetate buffer, 520 ml of 50 mM NaCl in Tris-acetate buffer, 350 ml of 100 mM NaCl in Tris-acetate buffer, 350 ml of 150 mM NaCl in Tris-acetate buffer and finally 270 ml of 200 mM NaCl in Tris-acetate buffer. The bioactive product was detected by bioassay (as described in Example 4c) and high pressure liquid chromotography (HPLC) during the 200 mM NaCl + Tris-acetate elution. The bioactive fraction was desalted using an ultrafiltration apparatus (Amicon DC-2) equipped with two 10K MW-cut off cartridges by diafiltering the fraction fifteen times against equal volumes of water. The desalted retentate was then lyophilized to dryness. The lyophilized powder (275 mg) was then dissolved in 0.05 M potassium phosphate buffer at pH 6.3 and injected in 4 equal portions onto an Altex Spherogel® TSK-3000 SW (small rigid spherical particles of bonded silica) column (21.5 mm x 60 cm length), and elution was followed by UV detection at 220 nm at 2 AUFS with a flow rate of 5 ml/min. The bioactive fraction was then desalted by dialyzing against water (2x2L) for 48 hrs using a 10K MW-cut off membrane. The desalted retentate was lyophilized to dryness to obtain about 45 mg of pure (> 95%) bioactive product. The homogeneity of the product (i.e., the purified active component of ATCC 53270) was determined by sodium dodecyl sulfate polyacrylamide gel electrophoresis (SDS-PAGE), HPLC, amino terminal amino acid analysis and isoelectric focusing.

## EXAMPLE 4

### CHARACTERIZATION OF THE PURIFIED
### ACTIVE COMPONENT OF ATCC 53270

The novel macromolecular tumor cell growth-inhibiting and cytotoxic antibiotic of the subject invention, AAC-345, has several distinguishing characteristics. Such characteristics include the following:

a) Nature

The purified active component of ATCC 53270 is a white amorphous powder. This characteristic was determined by visual observation.

b) Solubility

The antibiotic of the subject invention is soluble in water. This characteristic was determined by dissolving 1 mg of the purified active component of ATCC 53270 in 1 ml of water.

c) Stability

The antibiotic of the subject invention is sensitive to light in aqueous solution but is stable as a lyophilized powder. These characteristics were determined by the instability of active column fractions during the process of purification of the active component of ATCC 53270 described in Example 2. Such instability was judged by a bioactivity assay against B. subtilis which was performed by discing column fractions (25 µl in 6.35 mm disc) in an agar plate seeded with B. subtilis (ATCC 6633) and incubated at 37°C over night.

d) Molecular Weight

The antibiotic of the subject invention has a molecular weight of approximately 42,000 daltons.

This characteristic was determined by sodium dodecyl sulfate polyacrylamide gel electrophoresis (SDS - PAGE) using Laemmli 12.5% gel [See, Laemmli, Nature, 277, 680 (1970)].

e)    Isoelectric Point

The isoelectric point (pI) of the antibiotic of the subject invention is 2.9.  This characteristic was determined by utilizing the LKB 1818P Instruction Sheet for high performance analytical electrofocusing.

f)    Carbohydrate Content

The carbohydrate content of the antibiotic of the subject invention is 12%.  This characteristic was determined by the phenol/sulfuric acid method described in Methods in Enzymology, III, 93, (1957), Colowick et al. (Ed.), Academic Press, N.Y.

g)    High Pressure Liquid Chromatography (HPLC)

The antibiotic of the subject invention was eluted from an Altex Spherogel® TSK-3000 SW column (serial number 4K793 with guard column) (7.5 mm x 30 cm) by 0.05M potassium phosphate buffer (pH 6.3) using a Beckman Model 345/165 HPLC instrument.  After 11.5 minutes at a flow rate of 1 ml/min, peaks were detected at 220 nm and 280 nm at 0.1 AUFS.

h)    Extinction Coefficient

The antibiotic of the subject invention has an extinction coefficient ($E_1$cm 1% $H_2O$) of 21.6 at 220 nm ($UV_{\lambda max}$) and 3.0 at 275 nm ($UV_{\lambda max}$).  This characteristic was determined by dissolving 1 mg of the purified active component of ATCC 53270 in 1 ml of water and performing UV spectroscopy on a Beckman DU-7 spectro-photometer with a cell path length of 1 cm.

i)    Amino Acid Analysis

The antibiotic of the subject invention has the following amino acid content:

| Amino acid | Picomole |
|---|---|
| aspartic acid (asp) | 2862.4 |
| glutamic acid (glu) | 1524.4 |
| serine (ser) | 1135.8 |
| glycine (gly) | 1504.9 |
| histidine (his) | 41.3 |
| arginine (arg) | 107.4 |
| threonine (thr) | 2097.8 |
| alanine (ala) | 1765.7 |
| proline (pro) | 793.8 |
| tyrosine (tyr) | 134.5 |
| valine (val) | 1822.8 |
| methionine (met) | 223.9 |
| isoleucine (ile) | 456.3 |
| leucine (leu) | 307.0 |
| phenylalanine (phe) | 298.2 |
| lysine. (lys) | 49.4 |

The amino acid analysis was determined by using the "pico tag" method described in Water's Associates Operator's Manual #86746 (May 1984) and #88140 (June 1984).

j)    Partial Amino Acid Sequence

The antibiotic of the subject invention has the following partial amino acid coding sequence (beginning at the N-terminal): asp-thr-val-thr-val. Such partial sequence was determined by Edman degradation as described in the Operator's Manual of Model 470A Protein Sequencer (Applied Biosystems, Inc.).

0217621

## EXAMPLE 5

### PHARMACEUTICAL COMPOSITION

As a specific embodiment of a composition of this invention, the purified active component of ATCC 53270, prepared as described in Example 3, is dissolved in sterile aqueous solution and is administered parenterally in one dose of 10 mg/m$^2$ to a host animal afflicted with tumor cells sensitive to AAC-345.

Claims (BE, CH, DE, FR, GB, IT, LI, LU, NE, SE):

1.    A macromolecular tumor cell growth-inhibiting antibiotic which is produced by culturing ATCC 53270, or an active mutant or derivative thereof, in an aqueous nutrient medium containing assimilable sources of carbon and nitrogen under submerged aerobic conditions until a recoverable quantity of said antibiotic is produced and isolating said antibiotic from the fermentation broth produced by such culturing.

2.    The antibiotic of Claim 1 which is AAC-345.

3.    A crude broth of ATCC 53270 which is produced by culturing ATCC 53270, or an active mutant or derivative thereof, in an aqueous nutrient medium containing assimilable sources of carbon and nitrogen under submerged aerobic conditions until a recoverable quantity of AAC-345 is produced and isolating the crude broth from the fermentation broth produced by such culturing.

4.    An enriched crude broth of ATCC 53270 which is produced by culturing ATCC 53270, or an active mutant or derivative thereof, in an aqueous nutrient medium containing assimilable sources of carbon and nitrogen under submerged aerobic conditions until a recoverable quantity of AAC-345 is produced and isolating the enriched crude broth from the fermentation broth produced by such culturing.

5.    A macromolecular tumor cell growth-inhibiting antibiotic, AAC-345, which has the following characteristics:

| | |
|---|---|
| Nature: | White amorphous powder |
| Solubility: | Soluble in water |
| Stability: | Sensitive to light in aqueous solution, stable as lyophilized powder |
| Molecular Weight: | ~42,000 by SDS-PAGE |

Isoelectric Point:  pI = 2.9

Carbohydrate Content:  12% by phenol/sulfuric acid method

HPLC:  Retention time, 11.5 min; TSK-3000 SW Column (7.5 mm x 30 cm); Mobile Phase, 0.05M potassium phosphate buffer at pH 6.3; flow rate, 1 ml/min; detection, 220 nm and 280 nm

$UV_{\lambda max}$ ($E_{1 cm}^{1\%}$ in $H_2O$):  220 nm (21.6); 275 nm (3.0)

Amino Acid Analysis: (Picomole)  Asp (2862.4), Glu (1524.4), Ser (1135.8), Gly (1504.9), His (41.3), Arg (107.4), Thr (2097.8), Ala (1765.7), Pro (793.8), Tyr (134.5), Val (1822.8), Met (223.9), Ile (456.3), Leu (307.0), Phe (298.2), Lys (49.4).

Partial Amino Acid Sequence:  N-Asp-Thr-Val-Thr-Val-.

6.    A biologically pure culture of a microorganism which has the identifying characteristics of ATCC 53270, and active mutants and derivatives thereof.

7.    A pharmaceutical composition which comprises an effective, non-toxic, tumor cell growth-inhibiting amount of a macromolecular tumor cell growth-inhibiting antibiotic as defined in any one of claims 1, 2 or 5 and an inert pharmaceutically acceptable carrier or diluent.

8.    The composition of Claim 7 wherein the composition is in a dosage unit form adapted for parenteral administration.

9.    The composition of Claim 8 wherein the parenteral dosage unit is adapted to administer from about 1 to about 10 mg/m$^2$ of body surface.

10. A macromolecular tumor cell growth-inhibiting antibiotic according to any one of claims 1, 2 or 5, for use in inhibiting the growth of tumor cells in an animal.

11. A process for preparing a macromolecular tumor cell growth-inhibiting antibiotic as defined in any one of claims 1, 2 or 5 which process comprises culturing ATCC 53270, or an active mutant or derivative thereof, in an aqueous nutrient medium containing assimilable sources of carbon and nitrogen under submerged aerobic conditions until a recoverable quantity of said antibiotic is produced and isolating said antibiotic from the fermentation broth produced by such culturing.

12. A process for preparing a crude broth of ATCC 53270 which comprises culturing ATCC 53270, or an active mutant or derivative thereof, in an aqueous nutrient medium containing assimilable sources of carbon and nitrogen under submerged aerobic conditions until a recoverable quantity of AAC-345 is produced and isolating the crude broth from the fermentation broth produced by such culturing.

13. A process for preparing an enriched crude broth of ATCC 53270 which comprises culturing ATCC 53270, or an active mutant or derivative thereof, in an aqueous nutrient medium containing assimilable sources of carbon and nitrogen under submerged aerobic conditions until a recoverable quantity of AAC-345 is produced and isolating the enriched crude broth from the fermentation broth produced by such culturing.

Claims (Austria):

1.    A process for preparing a macromolecular tumor cell growth-inhibiting antibiotic which comprises culturing ATCC 53270, or an active mutant or derivative thereof, in an aqueous nutrient medium containing assimilable sources of carbon and nitrogen under submerged aerobic conditions until a recoverable quantity of said antibiotic is produced and isolating said antibiotic from the fermentation broth produced by such culturing.

2.    The process for preparing the antibiotic of Claim 1 which is AAC-345.

3.    A process for preparing a crude broth of ATCC 53270 which comprises culturing ATCC 53270, or an active mutant or derivative thereof, in an aqueous nutrient medium containing assimilable sources of carbon and nitrogen under submerged aerobic conditions until a recoverable quantity of AAC-345 is produced and isolating the crude broth from the fermentation broth produced by such culturing.

4.    A process for preparing an enriched crude broth of ATCC 53270 which comprises culturing ATCC 53270, or an active mutant or derivative thereof, in an aqueous nutrient medium containing assimilable sources of carbon and nitrogen under submerged aerobic conditions until a recoverable quantity of AAC-345 is produced and isolating the enriched crude broth from the fermentation broth produced by such culturing.

5.    A process according to claim 1 for preparing a macromolecular tumor cell growth-inhibiting antibiotic, AAC-345, which has the following characteristics:

Nature:                    White amorphous powder
Solubility:                Soluble in water

| Stability: | Sensitive to light in aqueous solution, stable as lyophilized powder |
| Molecular Weight: | ~42,000 by SDS-PAGE |
| Isoelectric Point: | pI = 2.9 |
| Carbohydrate Content: | 12% by phenol/sulfuric acid method |
| HPLC: | Retention time, 11.5 min; TSK-3000 SW Column (7.5 mm x 30 cm); Mobile Phase, 0.05M potassium phosphate buffer at pH 6.3; flow rate, 1 ml/min; detection, 220 nm and 280 nm |
| UV$\lambda_{max}$ (E$_{1\ cm}$ 1% in H$_2$O): | 220 nm (21.6); 275 nm (3.0) |
| Amino Acid Analysis: (Picomole) | Asp (2862.4), Glu (1524.4), Ser (1135.8), Gly (1504.9), His (41.3), Arg (107.4), Thr (2097.8), Ala (1765.7), Pro (793.8), Tyr (134.5), Val (1822.8), Met (223.9), Ile (456.3), Leu (307.0), Phe (298.2), Lys (49.4). |
| Partial Amino Acid Sequence: | N-Asp-Thr-Val-Thr-Val-. |

6.  A process according to any one of claims 1 to 5 wherein the culturing is conducted at a temperature between 25°C and 30°, at a pH between 7 and 8, for 10 to 100 hours.

7.  A process for preparing a pharmaceutical composition comprising bringing a macromolecular tumor cell growth-inhibiting antibiotic, as defined in any one of claims 1, 2 and 5 into association with a pharmaceutically acceptable carrier.

FIGURE 1 - FERMENTATION PROFILE OF ATCC 53270

0217621